(19)

Europäisches
Patentamt

European
Patent Office

Office européen
des brevets

(11) **EP 3 020 827 A1**

(12) **EUROPEAN PATENT APPLICATION**

(43) Date of publication:
18.05.2016 Bulletin 2016/20

(51) Int Cl.:
**C12Q 1/68** (2006.01)

(21) Application number: 14193074.3

(22) Date of filing: 13.11.2014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Carpegen GmbH**
**48149 Münster (DE)**

• **Systec Elektronik und Software GmbH**
**48161 Münster (DE)**

(72) Inventor: **Koltzscher, Max**
**48145 Münster (DE)**

(74) Representative: **von Renesse, Dorothea et al**
**König-Szynka-Tilmann-von Renesse**
**Patentanwälte Partnerschaft mbB**
**Mönchenwerther Str. 11**
**40545 Düsseldorf (DE)**

(54) **Method for determining a property of a starting sample**

(57) A method is provided for determining a property of a starting sample, for example the amount of the nucleic acid (DNA) present therein.

EP 3 020 827 A1

**Description**

The invention relates to a method for determining a property of a starting sample, for example the amount of the nucleic acid (DNA) present therein.

The polymerase chain reaction (PCR) is a method for amplifying DNA allowing even very small amounts of DNA to be detected. Said method comprises a respectively determined number of cycles composed of repeated heating and cooling of the reaction mixture for the melting and for the enzymatic replication of the sample DNA (target DNA) present therein. While the reaction is proceeding, the DNA produced in the respective prior cycle (or in the prior cycles) serves as matrix (template) for the replication of the DNA in the respective running cycle. This leads to an exponential multiplication (amplification) of the DNA, which in the ideal case doubles in each cycle.

The so-called real time PCR (or else quantitative PCR, qPCR) is characterized in that the accumulation of the PCR product (DNA) is detected during the running PCR, that is to say in real time. For this purpose, there is added to the reaction an initially inactive fluorescent dye which is made active by the DNA replication. The fluorescence is measured in each cycle - that is to say "in real time", and the amount of the amplified DNA can be inferred therefrom.

Consequently, the qPCR can be used to determine the number of the copies of the DNA originally present in the starting sample. The determination follows the general principle that the number of starting copies is greater the earlier a significant accumulation of the PCR product can be detected.

The monitoring of the accumulation of the PCR product is based as a rule on the detection of fluorescence radiation which is emitted after the excitation of the reaction mixture, and which is directly proportional to the amount of the PCR product.

PCR technologies, and among them precisely the qPCR, became established in the past in the area of point-of-care testing, in which diagnostic examinations are performed not in central laboratories, but in situ, for example in a hospital, a medical practice or pharmacy. Since the point-of-care diagnostics is also carried out in some circumstances by less fully trained staff, there is a need here for examination and evaluation techniques which are particularly noise-immune and as objective as possible.

It is known from Wiesner et al. (Nucl. Acids Res., .20, 5863-5864 (1992)) to evaluate the data of a polymerase chain reaction (PCR) by adopting the procedure that for each graphic representation of a result data set in which for a multiplicity of cycle values (n) a result value (y) is assigned to a respective cycle value (n) and then the initial concentration (c) in the sample and the amplification efficiency E is determined by linear regression of the result values (y) in the form of

$$\text{Log(result value)} = \text{Log(amplification efficiency)} \times \text{cycle value} + \text{Log(initial concentration c)}$$

($\log N_n = \log eff \cdot n + \log N_0$, in Wiesner's notation). This method does not work reliably in the case of very noisy measuring signals, in particular.

Against said background, it was the object of the invention to propose a method for determining a property of a starting sample which at least no longer has one of the disadvantages of the above prior art, or which at least proposes a method for determining a property of a starting sample which differs from the prior art.

The invention proceeds from the fundamental idea that in order to determine the property of the starting sample it is necessary to determine a data set from a result data set, and that particular importance attaches to the determination of the data set, in particular of the last cycle value of the data set, since the determination of the property can only be carried out unreliably with a data set which is too wide or too narrow in scope. In this case, in the result data set for a multiplicity of cycle values a result value is assigned to a respective cycle value, and in the data set for a plurality of cycle values a result value is assigned to a respective cycle value, the multiplicity of the cycle values included in the result data set being greater than the plurality of the cycle values included in the data set.

The invention likewise proceeds from the finding that measuring errors included in the result values must be taken into account when determining the properties of the starting sample. For this reason, one embodiment of the invention provides to use a curve fitting to calculate from the data set the parameters of a function which expresses a result value as a function of the cycle value, the function containing an exponential term in which an initial value (c) features as parameter, and in which the cycle value features as exponent (n), it having been recognized according to the invention that said function has a system term added to the exponential term. According to said embodiment, it is assumed in the invention that the property to be determined can be determined on the basis of the parameter found for the initial value (c) in the curve fitting.

The invention is thereby distinguished from the procedural approach of Wiesner et al. (Nucl. Acids Res., .20, 5863-5864 (1992)) since it was recognized there only that a data set can be expressed by the function $\log N_n = \log eff \cdot$

$n + \log N_0$, that is to say the logarithm of a function which expresses a result value ($N_n$) as a function of the cycle value (n), the function including, before being logarithmized, an exponential term in which an initial value ($N_0$) features as parameter, and in which the cycle value (n) features as exponent. However, said procedural approach completely neglects the influences resulting from the system for producing the data set, and is therefore not suitable for being used as automatically as possible. Such a very largely automatic, and therefore reliable, approach is desired precisely for a point-of-care diagnostics based on a PCR.

According to the invention, it has been recognized that the influences of the system for producing the data set are best expressed by a system term added to the exponential term. Only if the influence of the system and the exponential growth are accounted for jointly in the curve fitting, a value for the initial value (c) can be realiably generated on the basis of which the property to be determined can be reliably output.

The method according to the invention uses a data set in which a result value is assigned to a respective cycle value for a plurality of cycle values. Preferably it is concerned in this case with a data set in which the plurality of cycle values is a sequence of natural numbers, in particular a sequence of natural numbers in the case of which one natural number is followed as next cycle value by the next natural number in the succession of the natural numbers (for example "1, 2, 3, 4, 5, ..." or "3, 4, 5, 6, 7, 8, ...").

In the majority of the applications of the invention, and thus in accordance with a preferred embodiment, the data set consists of a series of value pairings comprising a cycle value and the result value respectively assigned to it, to which the sequence of the value pairings within the data set results from the sequential order in which measured values for producing result values are recorded. In a particularly preferred embodiment, the second value pairing, respectively following as next a first value pairing within the sequence, is that value pairing which is produced from the measured values which were recorded as next after the measured values which were recorded to produce the first value pairing.

In the case of the qPCR, the result value is preferably the fluorescence intensity y measured at the end of a respective cycle. Said fluorescence intensity is usually represented dimensionless in arbitrary units.

The method according to the invention carries out a curve fitting on the data set. Curve fittings are also sometimes termed compensating computation, compensation, parameter estimation, matching, regression or fit(ting). In a preferred embodiment, the method of least squares is used as curve fitting.

In accordance with the inventive method, the curve fittings carried out on the data set in order to employ the data of the data set to determine the parameters of a function which expresses a result value as a function of the cycle value, the function including an exponential term in which an initial value (c) features as parameter, and in which the cycle value features as exponent, and the function has a system term added to the exponential term. Said function can also be expressed as follows

$$F(n) = A + c\,E^n,$$

F(n) being the value calculated by the function that is to correlate with the result value relating to the respective cycle value (n), A being the system term, and $cE^n$ being the exponential term in which an initial value (c) features as parameter and in which the cycle value (n) features as exponent. In a preferred embodiment, the parameter E of the exponential term can be a measure of the efficiency of the amplification.

In a preferred embodiment, the system term A can be expressed by the relationship $A = a + b \cdot n$. In this case, the parameter (a) can be understood as a measure of the mean value of the background noise in the production of the result values. The parameter (b) can in a prefered embodiment, be used for the purpose of describing the effect that the result values produced could become smaller or larger towards the beginning.

In a preferred embodiment, a curve fitting is therefore carried out on the data set in order to use the data of the data set to determine the parameters of a function which includes at least the term $a + b \cdot n + cE^n$. Particularly preferred is a curve fitting on the data set with the aim of using the data of the data set to determine the parameters of a function which can be expressed by $a + b \cdot n + cE^n$.

In a preferred embodiment, the term A that in one preferred embodiment is chosen to be a linear term ($a + b \cdot n$) can be supplemented by further terms, in order, for example, to form a polynomial of nth degree. It is thereby possible to determine the target parameter (c) yet more precisely in the course of the curve fitting.

The parameter found for the initial value (c) in the curve fitting can directly represent the property to be determined. In a preferred embodiment, in order to output the property to be determined the parameter found for the initial value (c) in the curve fitting is multiplied by a system-specific factor s. If, for example, the original DNA amount ($Q_e$) of a sample is targeted as property to be determined, said amount can be determined using the formula $Q_e = s * c$. The system-specific factor s can easily be determined with the aid of a reference sample whose original DNA amount is known, and is carried out using the method according to the invention in order to use the formula $Q_e/c = s$ with the aid of the parameter c thus determined and the original DNA amount ($Q_e$) known for the reference sample to determine the factor s. The factor s can also be used to express the parameter in the units desired, for example in a PCR the amount of DNA in the original sample can be expressed dimensionless as number of copies or as concentration, for example as g/l or mol/l.

In an alternative embodiment the property of the starting sample is obtained on the basis of further calculations. Having identified the system term A in the manner described above, it is possible to create a modified data set by subtracting from each result value in the data set the value of the system term A applicable for the cycle value of that result value. If the system term A found is a constant, this constant is substracted from each result value of the data set to create the modified data set. If the system term A is a function of the cycle value, for example if $A = a + b \cdot n$, from each result value the system term A applicable for the cycle value corresponding to this result value is substracted from the result value to create a modified result value that is to be part of the modified data set. If the modified data set was plotted, the plotted points would be much closer to a clean exponential curve than if the data set was plotted, because the data in the modified data set has been freed from any ambigouties that stem from the system. This exponential curve could be expressed as $F(n) = c\, E^n$. In this alternative embodiment, the initial value (c) could be determined by carrying out a curve fitting on parts of the modified data set, for example a curve fitting to the expression $F(n) = c\, E^n$. Alternatively a logarithmic data set can be created from the modified data set. To create the logarithmic data set, the logarithm of each modified result value is taken to create a logarithmic result value. For example the natural logarithm or a logarithm to base 2 or a logarithm to base 10 or to any other suitable base can be taken. A graphic representation of parts of the logarithmic data set would be approximately a straight line ($\ln (c\, E^n) = n \cdot \ln (E) + \ln (c)$). In performing a curve fitting of the logarithmic result value to the expression $n \cdot \ln (E) + \ln (c)$), the initial value (c) can be found from the point of intersection with the ordinate.

In this alternative embodiment the curve fitting for a straight line that best fits the logarithmic data set, the method of least squares can be used. In one possible embodiment, for a preset number of successive values, for example for four successive values, a curve fitting for a straight line can be performed on these values. The standard deviation from the points to the fitted straight line can be determined. In such an approach the set of successive values and the line fitted to this set of values is chosen that provides the minimum standard deviation. The initial value (c) is taken from the point of intersection with the ordinate for this line. To further enhance the quality of the resulting initial value, it can be additionally be required that the line is only fitted to those successive values that all have a parameter E calculated in the approach discussed above that lies between a predetermined value $E_{min}$ and a predetermined value Emax. This way, it is made clear that the curve fitting is only conducted for values from the exponential phase. In addition (to verify the results) or as an alternative to determining the standard deviation, for all sets of successive values that have been looked at and all sets of straight lines that have been created thereby, the straight line with the maximum inclination is chosen. The initial value (c) is taken from the point of intersection with the ordinate for this line. To further enhance the quality of the resulting initial value, it can be additionally be required that the line is only fitted to those successive values that all have a parameter E calculated in the approach discussed above that lies between a predetermined value $E_{min}$ and a predetermined value $E_{max}$ and provides a standard deviation that is below a predetermined value. This way, it is made clear that the curve fitting is only conducted for values from the exponential phase.

In a PCR method, during a cycle the DNA-containing reaction mixture (sample) is heated in a system. Typical PCR-suitable systems are well known to the person skilled in the art, an example being the so-called thermocycler. The double strands of the DNA are melted owing to being heated to a specific value. In order subsequently to enable the hybridization of the primers present in the reaction mixture to the single strands (*annealing*), the sample is cooled down to a temperature suitable therefor. This is usually performed automatically by the system. Thereupon, primers are elongated by a polymerase present in the reaction mixture (DNA replication). For this purpose, as well, the reaction mixture may be brought up to a suitable temperature by the system. This results in new double strands (*amplification*). A cycle is thereby terminated. A new cycle begins with a renewed heating of the system in order to melt the DNA.

In real time PCR (qPCR), there is added to the reaction mixture a reagent which indicates the amplification of the DNA by a fluorescence signal. Said fluorescence signal can be used, for example, as result value of the cycle. Possible reagents are familiar to the person skilled in the art, one example being SYBR green, which is intercalated into the amplified, and thus then double-stranded, DNA, and then fluoresces. However, by way of example it is also possible to add to the reaction mixture a quencher which firstly quenches the fluorescence and is removed upon amplification. Only then is it possible to detect the fluorescence. Another example are hydolysis probes, which can be used to generate a fluorescence signal.

Thus, a PCR method can be used to produce a result data set in which a result value is assigned to a respective cycle value for a multiplicity of cycle values. In a preferred embodiment, the data set to be used for the method according to the invention is produced from the result data set. The multiplicity of the cycle values included in the result data set is therefore greater than the plurality of the cycle values included in the data set. In a preferred embodiment, the sequence of the cycle values of the data set is a subgroup of a sequence of cycle values of the result data set.

In one embodiment, the method according to the invention provides that the sequence of the cycle values of the data set is a subgroup of the sequence of the cycle values of the result data set, and the last result value of the sequence of cycle values of the data set is determined as follows: proceeding from a starting cycle value of the result data set for a plurality of consecutive sequences of three cycle values which are respectively denoted as first cycle value, second cycle value and third cycle value within the respectively considered sequence in accordance with their sequential order in the result data set, the sequence is determined, in the case of which

- the result value assigned to the second cycle value of the considered sequence is greater than the result value assigned to the first cycle value of the considered sequence, and
- the curvature value (z), which results from the sum of the result value assigned to the first cycle value and of the result value assigned to the third cycle value of the considered sequence and the subtraction of twice the result value assigned to the

second cycle value of the considered sequence, assumes the highest value among the considered sequences,

the second cycle value of the sequence which satisfies said requirements being used as last cycle value of the data set.

In the plurality of the areas of use of the invention, it is to be expected that the result values increase exponentially, at least for a subgroup of cycle values with increasing cycle values. It has been recognized that the property of the starting sample can be determined particularly well if the data set to be evaluated includes only the cycle values (and the result values assigned thereto) up to the (and including the) cycle value from which the result values no longer increase exponentially, but the profile of the result values flattens off. In this case, an attempt should be made to determine said last cycle value with particular accuracy. Specifically, if a curve fitting is carried out with the aid of the data set in order to determine the targeted property, the addition of even one further cycle value (and of the result value assigned to it) can lead to a substantial variation in the parameters determined in the curve fitting. It has been recognized in this case that the last cycle value of the data set can be determined particularly reliably by the method of Claim 1.

In the preferred embodiment, where the curvature value (z) is determined, it is possible in a yet even more preferred embodiment to stop the search for the sequence when the curvature value (z) becomes zero or alternatively to stop the search for the sequence either when the curvature value (z) becomes negative. In cases where the curvature value (z) becomes zero or becomes negative, the curvature of a curve that previously had been exponential has changed into a curve that flattens out. This can save having to perform further cycles and can thus reduce the time necessary to perform the determination of the initial value.

It is conceivable for the method according to the invention that for the initial cycle value of the result data set, which then also becomes the initial cycle value of the data set, use is made of the first result value, that is to say, for example, the measured value of the amplification determined when a cycle according to the PCR method is first carried out on the starting sample. However, it is also possible to conceive embodiments in the case of which a later cycle value of the result data set is used as initial cycle value of the data set. This procedure permits the exclusion of initial inaccuracies in the determination of the result value, which can occur, above all, in the first cycle of a PCR method carried out on a starting sample. For example, the third cycle can be used as initial cycle of the data set.

In a preferred embodiment, a maximum cycle value can be predetermined ($N_R$). Said maximum cycle value can be used in order to check whether errors have occurred in preparing the data set from the result data set. For example an error message can be generated if the predetermined maximum cycle value has been reached without the curvature value (z) becoming zero or negative. By way of example, the numeral 40 can be selected as maximum cycle value.

Furthermore, it is possible to fix a minimum number of cycle values. This can also be used in order to check whether errors have occurred in preparing the data set from the result data set. If it is established that the data set includes fewer cycle values than the minimum cycle value, it is possible in a preferred embodiment of the method according to the invention to generate advice for an operator.

In a preferred embodiment, a minimum value $E_{min}$ can be fixed for the parameter E in the formulae reproduced. Should it emerge that a value which is below the value $E_{min}$ is determined in the curve fitting for the parameter E, it is possible to output a warning.

In a preferred embodiment, a maximum value $E_{max}$ can be fixed for the parameter E in the formulae reproduced. Should it emerge that a value which is above the value $E_{max}$ is determined in the curve fitting for the parameter E, it is possible to output a warning.

In a preferred embodiment, a minimum result value difference $\Delta y_{max}$ can be fixed. If the difference between the result value of the last cycle value of the data set and the result value of the initial cycle value of the data set is smaller than said minimum result value difference $\Delta y_{max}$, it is possible to output a warning.

In a preferred embodiment, it is possible to output a warning when the parameter to be determined for the initial value (c) is less than zero.

The method according to the invention can be used to evaluate any desired form of PCR in which the amplification of the DNA present in the reaction mixture can be traced via a signal, in particular via a fluorescence signal. The only assumption is that a result data set with a multiplicity of cycle values is produced in which a respective cycle value can be assigned a result value. In this case, the starting sample can be a DNA, or else another form of nucleic acid (for example a RNA) which is subsequently transcribed into a corresponding DNA using methods familiar to the person skilled in the art. An example of this is the use of RNA, which can be transcribed into DNA by a reverse transcriptase and then be quantified via a real time PCR. The real time PCR produces the result data set required for the method according to the invention.

The method according to the invention is suitable, in particular, for use in PCR systems to be utilized in point-of-care systems. It is precisely here that, taking account of the particularities of the system, the better fitting rendered possible by the invention, and the better representation of the measured value profile identified by the invention, afford a rise in the reliability of the evaluations carried out with point-of-care systems in a fashion divorced from the qualification of the operator.

The invention is explained in more detail below with the aid of a drawing showing only one exemplary embodiment of the invention. In the drawing

Figure 1    shows a graph which represents a result data set and a function whose parameters are determined by a curve fitting on a data set produced from the result data set and

Figure 2    shows a graph which represents a logarithmic result data set and a straight line fitted to four successive values of the logarithmic data set.

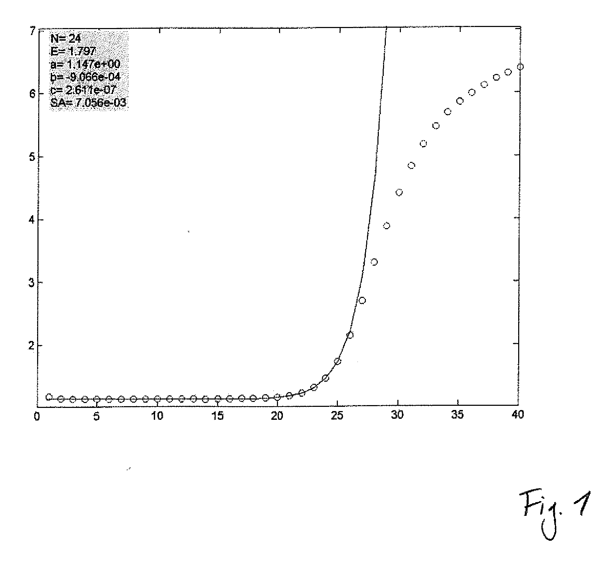

The graph illustrated in Figure 1 is a graphic representation of a result data set. The result data set includes a multiplicity of cycle values, specifically the sequence of the natural numbers 1 to 40. The graph illustrates the result value assigned to the respective cycle value of the result data set. The result value is the fluorescence intensity y determined at the end of the respective cycle.

A data set in which a result value is assigned to a respective cycle value for a plurality of cycle values was produced from the result data set. The sequence of the cycle values of the data set is a subgroup of the sequence of the cycle values of the result data set. As initial cycle value, the data set includes the cycle value 1, and as last cycle value the cycle value 24 (n = 24).

The last cycle value of the sequence of cycle values of the data set was determined as follows: proceeding from the starting cycle value (n = 1) of the result data set for a plurality of consecutive sequences of three cycle values (n = 1, 2, 3; n = 2, 3, 4; n = 3, 4, 5; n = 4, 5, 6, etc.) which are respectively denoted as first cycle value (as an example n = 4), second cycle value (in the example n = 5) and third cycle value (in the example n = 6) within the respectively considered sequence (for example n = 4, 5, 6) in accordance with their sequential order in the result data set, the sequence is determined in the case of which

- the result value assigned to the second cycle value of the considered sequence is greater than the result value assigned to the first cycle value of the considered sequence, and
- the curvature value z, which results from the sum of the result value, assigned to the first cycle value and of the result value assigned to the third cycle value of the considered sequence, and the subtraction of twice the result value assigned to the second cycle value of the considered sequence, assumes the highest value among the considered sequences.

The second cycle value of the sequence which satisfies said requirements is used as last cycle value of the data set, here the cycle value n = 24.

Figure 1 also includes the graphic illustration of a function. The function is

$$f(n) = a + bn + cE^n = A(n) + cE^n$$

The parameters (a), (b), (c), E of the function were implemented on the data set in the course of a curve fitting. In this case, the parameters reproduced in the figure (top left) were likewise determined.

The property to be determined, specifically the number of the molecules in the starting sample molar, can be determined from the parameter 2.611e-07 thus determined for the initial value c.

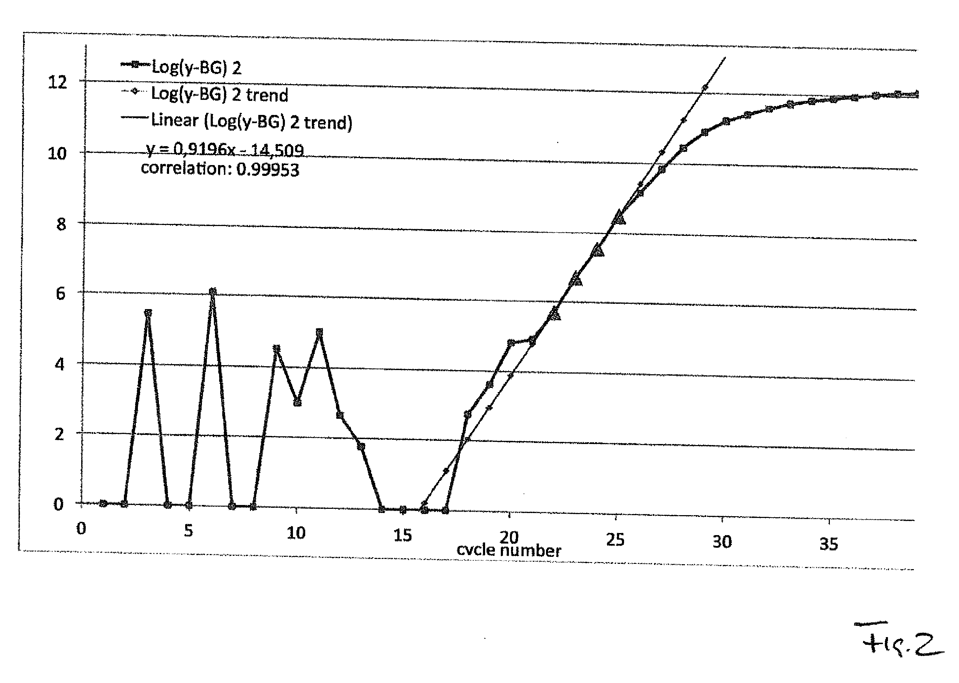

Fig. 2 shows the graphic representation of a logarithmic data set. Having identified the system term A(n) in the manner described above, a modified data set is created by subtracting from each result value in the data set the value of the system term A(n) applicable for the cycle value of that result value. From the modified data set, the logarithmic data set is created by taking the logarithm to base 2 of each modified result value. As can be seen from Fig. 2 a graphic representation of parts of the logarithmic data set would be approximately a straight line (In (c $E^n$) = n • In (E) + In (c)), while other parts show varying values. In performing a curve fitting of the logarithmic result value to the expression n • In (E) + In (c)) on these parts, the initial value (c) can be found from the point of intersection with the ordinate.

In this alternative embodiment the curve fitting for a straight line that best fits the logarithmic data set, the method of least squares is used. In one possible embodiment, for four successive values, a curve fitting for a straight line can be performed on these values. The standard deviation from the points to the fitted straight line can be determined. In such an approach the set of successive values and the line fitted to this set of values is chosen that provides the minimum standard deviation. The initial value (c) is taken from the point of intersection with the ordinate for this line. As can be seen from Fig. 2, for the first cycle values, the logarithmic result values of four consecutive values can only be fitted to a straight line with a large standard diviation. However, in the graphic representation of the logarithmic data set, there is a characteristic series of values corresponding to the exponential phase that can be seen in Fig. 1. In this characteristic series of values, the values in Fig. 2 can be approximated with a straight line. To determine the value (c) that series of four values is chosen that has a straight line fitted to it with the least standard deviation and the value (c) is taken from the intersection of this straight line with the ordinate.

In addition (to verify the results) or as an alternative to determining the standard deviation, for all sets of successive values that have been looked at and all sets of straight lines that have been created thereby, the straight line with the maximum inclination is chosen. To further enhance the quality it is required that the line is only tilted to those successive values that have a parameter E calculated in the approach discussed above that lies between a predetermined $E_{min}$ and a predetermined $E_{max}$ and provides a standard deviation that is below a predetermined value. The initial value (c) is taken from the point of intersection with the ordinate for this line.

## Claims

1.    Method for determining a property of a starting sample, in which

   - the starting sample is amplified in a system in a first cycle, and a measure of the amplification is acquired as a result value,
   - the amplification in the system in a respecitve cycle and the acquisition of a measure of the amplification as a result value for the respective cycle is repeated at least n times, and each cycle is assigned a cycle value for identification,
   - so that a result data set is produced in which a result value is assigned to a respective cycle value for a multiplicity of cycle values,

   there being produced from the result data set a data set in which a result value is assigned to a respective cycle value for a plurality of cycle values, the multiplicity of the cycle values included in the result data set being greater than the plurality of the cycle values included in the data set,
   the sequence of the cycle values of the data set being a subgroup of the sequence of the cycle values of the result data set, and the last cycle value of the sequence of cycle values of the data set being determined as follows: proceeding from a starting cycle value of the result data set for a plurality of consecutive sequences of three cycle values which are respectively denoted as first cycle value, second cycle value and third cycle value within the respectively considered sequence in accordance with their sequential order in the result data set, the sequence is determined, in the case of which

   - the result value assigned to the second cycle value of the considered sequence is greater than the result value assigned to the first cycle value of the considered sequence, and
   - the curvature value z, which results from the sum of the result value, assigned to the first cycle value and of the result value assigned to the third cycle value of the considered sequence and the subtraction of twice the result value assigned to the second cycle value of the considered sequence assumes the highest value among the considered sequences,

   the second cycle value of the sequence which satisfies said requirements being used as last cycle value of the data set.

2.    Method for determining a property of a starting sample, in which

   - the starting sample is amplified in a system in a first cycle, and a measure of the amplification is acquired as a result value,

- the cycle is repeated at least n times, and each cycle is assigned a cycle value for identification,

so that a data set is produced in which a result value is assigned to a respective cycle value for a plurality of cycle values, wherein

- a curve fitting is carried out on the data set in order to use data of the data set to determine the parameters of a function which expresses a result value as a function of the cycle value, the function containing an exponential term in which an initial value (c) features as parameter, and in which the cycle value features as exponent, and the function has a system term added to the exponential term, and
- a property to be determined is output on the basis of the parameter found for the initial value (c) in the curve fitting.

3.  Method according to Claim 2, **characterized in that** the data set is produced from a result data set, in the result data set for a multiplicity of cycle values a result value being assigned to a respective cycle value, the multiplicity of the cycle values contained in the result data set being greater than the plurality of the cycle values contained in the data set.

4.  Method according to Claim 3, **characterized in that** the sequence of the cycle values of the data set is a subgroup of the sequence of the cycle values of the result data set, and the last cycle value of the sequence of cycle values of the data set is determined as follows: proceeding from a starting cycle value of the result data set for a plurality of consecutive sequences of three cycle values which are respectively denoted as first cycle value, second cycle value and third cycle value within the respectively considered sequence in accordance with their sequential order in the result data set, the sequence is determined, in the case of which

- the result value assigned to the second cycle value of the considered sequence is greater than the result value, assigned to the first cycle value of the considered sequence and
- the curvature value z, which results from the sum of the result value assigned to the first cycle value and of the result value assigned to the third cycle value of the considered sequence and the subtraction of twice the result value assigned to the second cycle value of the considered sequence assumes the highest value among the considered sequences, the second cycle value of the sequence which satisfies said requirements being used as last cycle value.

5.  Method according to one of Claims 2 to 4, **characterized in that** the method of least squares is used to carry out the curve fitting.

6.  Method according to one of Claims 1 to 5, **characterized in that** the starting sample is a DNA and the result value is a measure of a fluorescence intensity.

7.  Use of a method according to one of Claims 1 to 6 in PCR systems for use in point-of-care systems.

N= 24
E= 1.797
a= 1.147e+00
b= -9.066e-04
c= 2.611e-07
SA= 7.056e-03

Fig. 1

Fig. 2

Europäisches
Patentamt

European
Patent Office

Office européen
des brevets

# EUROPEAN SEARCH REPORT

Application Number

EP 14 19 3074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/029924 A2 (STRATAGENE INC [US]; TAYLOR ROGER H [US]; ORR ALAN R [US]) 10 April 2003 (2003-04-10) * page 16, line 22 - line 29; claims 1-4 * | 1-7 | INV. C12Q1/68 |
| X | EP 2 163 999 A2 (ROCHE DIAGNOSTICS GMBH [DE]; HOFFMANN LA ROCHE [CH]) 17 March 2010 (2010-03-17) * paragraph [0041]; figure 9 * * paragraph [0026]; claim 1 * | 1-7 | |
| A | US 2006/009916 A1 (LI XITONG [US] ET AL) 12 January 2006 (2006-01-12) * paragraph [0088] * | 1 | |
| A | EP 1 138 783 A2 (HOFFMANN LA ROCHE & CO AG F) 4 October 2001 (2001-10-04) * paragraph [0025] - paragraph [0026] * * paragraph [0025] * | 1 | |

TECHNICAL FIELDS
SEARCHED (IPC)

C12Q

The present search report has been drawn up for all claims

| Place of search | Date of completion of the search | Examiner |
|---|---|---|
| The Hague | 19 May 2015 | Gabriels, Jan |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

 

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 14 19 3074

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2015

| Patent document cited in search report | | Publication date | Patent family member(s) | | Publication date |
|---|---|---|---|---|---|
| WO 03029924 | A2 | 10-04-2003 | CA | 2462823 A1 | 10-04-2003 |
| | | | EP | 1438430 A2 | 21-07-2004 |
| | | | JP | 2005504543 A | 17-02-2005 |
| | | | US | 2003148332 A1 | 07-08-2003 |
| | | | WO | 03029924 A2 | 10-04-2003 |
| EP 2163999 | A2 | 17-03-2010 | CA | 2678235 A1 | 12-03-2010 |
| | | | CN | 101710363 A | 19-05-2010 |
| | | | EP | 2163999 A2 | 17-03-2010 |
| | | | JP | 5558762 B2 | 23-07-2014 |
| | | | JP | 2010081937 A | 15-04-2010 |
| | | | US | 2010070185 A1 | 18-03-2010 |
| US 2006009916 | A1 | 12-01-2006 | US | 2006009916 A1 | 12-01-2006 |
| | | | WO | 2006014509 A2 | 09-02-2006 |
| EP 1138783 | A2 | 04-10-2001 | AT | 370248 T | 15-09-2007 |
| | | | DE | 60129869 T2 | 08-05-2008 |
| | | | EP | 1138783 A2 | 04-10-2001 |
| | | | ES | 2291238 T3 | 01-03-2008 |
| | | | JP | 3589638 B2 | 17-11-2004 |
| | | | JP | 2001314195 A | 13-11-2001 |
| | | | US | 2003165832 A1 | 04-09-2003 |
| | | | US | 2004153254 A1 | 05-08-2004 |
| | | | US | 2010021923 A1 | 28-01-2010 |
| | | | US | 2012270222 A1 | 25-10-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WIESNER et al.** *Nucl. Acids Res.,* 1992, vol. 20, 5863-5864